# EUROPEAN PATENT SPECIFICATION

(11) **EP 1 178 756 B1**
(45) Date of publication and mention of the grant of the patent: **22.11.2006**
(21) Application number: 00912165.8
(22) Date of filing: 03.03.2000
(51) Int. Cl.: A61B 17/02

(54) **DEVICE FOR STABILIZING A TREATMENT SITE**
VORRICHTUNG ZUR STABILISIERUNG EINER BEHANDLUNGSSTELLE
DISPOSITIF DE STABILISATION D'UNE ZONE DE TRAITEMENT

(30) Priority: 16.04.1999 US 293334
(43) Date of publication of application: 13.02.2002
(73) Proprietor: Medivas, LLC, La Jolla, CA 92037 (US)
(72) Inventor: TAKAHASHI, Masao, M.D., Chigasaki, 253-0054 (JP); EDELMAN, Elazer, E., Brookline, MA 02445 (US); CARPENTER, Kenneth, W., La Jolla, California 92037 (US)
(74) Representative: Schmidt, Josef Heinrich
(86) International application number: PCT/US2000/005563
(87) International publication number: WO 2000/062680

(56) References cited:
- WO-A-97/10753
- WO-A-98/37814
- DE-U- 9 004 513
- US-A- 5 171 254
- US-A- 5 782 746
- US-A- 5 885 271

## Description

### FIELD OF THE INVENTION

The present invention generally relates to surgery on body tissues and organs. More specifically, the present invention relates to an apparatus for temporarily immobilizing a local area of tissue subject to motion, such as the heart wall, which permits a treatment procedure to be performed on that local area of tissue.

### BACKGROUND OF THE INVENTION

Coronary artery disease remains the leading cause of morbidity and mortality in Western societies and is manifested in a number of ways. For example, disease of the coronary arteries can lead to insufficient blood flow to various areas of the heart. This can lead to the discomfort of angina and the risk of ischemia. In severe cases, acute blockage of coronary blood flow can result in irreversible damage to the myocardial tissue, including myocardial infarction and the risk of death.

A number of approaches have been developed for treating coronary artery disease. In less severe cases, it is often sufficient to merely treat the symptoms, with pharmaceuticals, or treat the underlying causes of the disease, with lifestyle modification. In more severe cases, the coronary blockage can be treated endovascularly or percutaneously using techniques such as balloon angioplasty, atherectomy, laser ablation, stents, and the like.

In cases where these approaches have failed or are likely to fail, it is often necessary to perform a coronary artery bypass graft procedure. This procedure generally involves opening the chest by median sternotomy, spreading the left and right rib cage apart; and opening the pericardial sac to achieve direct access to the heart. Next, a blood vessel or vessels for use in the graft procedure are mobilized from the patient. This usually entails mobilizing either a mammary artery or a saphenous vein, although other graft vessels may also be used.

Commonly, a heart-lung or cardiopulmonary bypass is performed so that the beating of the heart can be stopped during the surgical procedure. This usually entails arterial and venous cannulation, connecting the bloodstream to a heart-lung machine, cooling the body to about 32 degrees Celsius, cross-clamping of the aorta and cardioplegic perfusion of the coronary arteries to arrest and cool the heart to about 4 degrees Celsius. The arrest or stoppage of the heart is generally required because the constant pumping motion of the beating heart would make surgery upon the heart difficult in some locations and extremely difficult if not impossible in other locations

Once cardiac arrest is achieved, a graft (or grafts) is attached to the relevant portions of a coronary artery (or arteries) followed by weaning from the cardiopulmonary bypass, restarting the heart, and decannulation. Finally the chest is closed.

However, use of the cardiopulmonary bypass may create difficulties for the patient and increase the expense and time required for the procedure. In a cardiopulmonary bypass, all the patient's blood, which normally returns to the right atrium, is diverted to a system which supplies oxygen to the blood, removes carbon dioxide, and returns the blood, at sufficient pressure, into the patient's aorta for further distribution into the body. Generally, such a system requires several separate components, including an oxygenator, several pumps, a reservoir, a blood temperature control system, filters, and flow, pressure, and temperature sensors.

Problems may develop during cardiopulmonary bypass due to biological reaction of the blood to non-endothelially lined surfaces, i.e. surfaces unlike those of a blood vessel. In particular, exposure of blood to foreign surfaces results in the activation of virtually all the humoral and cellular components of the inflammatory response, as well as some of the slower reacting specific immune responses. Other complications from cardiopulmonary bypass include loss of red blood cells and platelets due to shear stress damage. In addition, cardiopulmonary bypass requires the use of an anticoagulant, such as heparin. The anticoagulant may, in turn, increase the risk of hemorrhage. Finally, cardiopulmonary bypass sometimes necessitates administering additional blood to the patient. The additional blood, if from a source other than the patient, may expose the patient to blood borne diseases.

Due to the risks incurred during cardiopulmonary bypass, others have attempted to perform a coronary artery bypass graft procedure without cardiac arrest and cardiopulmonary bypass in a procedure known as an "off pump coronary artery bypass" (OPCAB) procedure. For example, Trapp and Bisarya (*Annals Thorac. Surg.* 19(1):1-9, 1975), immobilized the area of the bypass graft by encircling sutures deep enough to incorporate enough muscle to suspend an area of the heart while preventing damage to the coronary artery. More recently, Fanning *et al (Annals Thorac. Surg.* 55: 486-489, 1993) reported immobilizing the area of the bypass graft with stabilization sutures.

While these attempts have achieved some success, they generally require enhanced skill of the surgeon to properly create the anastomosis because, even with use of sutures to suspend a portion of the surface of the heart upon which the surgery is conducted, the beating heart continues to move in the relevant area more than desired. In addition, the sutures may cause a myocardial tear, an injury of the coronary artery branches, or such complications as embolism or focal arteriosclerosis resulting from the pressures of the ligatures upon the artery.

In order to solve such problems associated with the use of sutures to stabilize the site of an anastomosis upon the surface of a beating heart, a device known as a "local myocardial compression device" has been developed wherein myocardial portions on both sides of the coronary artery on which anastomosis is to be performed are compressed with two-tined fork-like instrument to apply pressure upon the artery and the heart itself so as to stabilize the treatment site. While use of this device has met with some success, the application of local compression to the heart can effect considerable local deterioration of cardiac function, particularly when cardiopulmonary bypass is not used to supplement blood circulation. In addition, this device does not address the problem of bleeding from a locally dissected coronary artery intended for anastomosis.

To address the undesirable effect of compression of the heart, such as is caused by use of the local myocardial compression device, a suction-assisted device has been developed having two paddles, each of which includes a series of suction ports located at the point where the device interfaces with the surface of the heart as described in U. S. Patent No. 5,836,311. The paddles are applied to the surface of the heart across an arterial section intended as an anastomotic site and suction applied through the suction ports is employed to lift and hold the surface tissue of a beating heart at the anastomotic site to minimize motion of the treatment site while the heart continues to beat underneath. This device may be used in either a conventional, open-chest environment or in an endoscopic minimally invasive procedure. However, it has been discovered that application of pressure at localized points using such a device can cause suction induced hemorrhages on the surface of the heart that result in scarring of the heart.

The need for stabilization of a moveable surgical or biopsy site (i.e., a treatment site) is not limited to the case of the beating heart. Endoscopic catheters, for example biopsy and angiogenesis catheters, are known for use in connection with procedures involving removal of small tissue samples or injection of therapeutic drugs or genetically altered structures, such as for use in gene therapy. For accuracy of results, it is important that the depth of the tissue sample or injection be precisely controlled. When such endoscopic catheters are used at interior areas of the body that are subject to movement, such as the bowel or stomach, the lungs and the diaphragm, it is especially difficult to assure that the injection or tissue sampling device penetrates the target tissue to a desired or uniform depth. Such procedures could be facilitated by use of a device adapted for use at the tip of an endoscopic catheter that helps to stabilize the treatment site during such a therapeutic or diagnostic procedure.

WO 98 37814 discloses a device to hold an anastomotic site of coronary artery motionless and bloodless for the bypass operation. The device includes a suction body having a flexible channel, which defines an outer surface and an inner surface of the device. The inner surface forms an opening for exposing the anastomotic site.

Thus, there is a need in the art for new and better devices useful for stabilizing a surgical site, such as the surface of the beating heart, or for endoscopically stabilizing a an interior therapeutic or diagnostic treatment site.

### SUMMARY OF THE INVENTION

In spite of the fact that off-pump coronary artery bypass surgery undertaken on the patient's beating heart (OPCAB) usually is successful with quick recovery of the patient and speedy return to work, many cardiovascular surgeons hesitate to perform such surgery because it requires an extremely highly-advanced skill and a special talent for avoiding the accompanying risks of complications to the coronary artery. It is an object of the present invention to provide a device to securely hold a surgical site, such as an anastomotic site on a coronary artery, relatively motionless throughout a bypass operation so that even surgeons having ordinary cardiovascular surgical skills can undertake such surgery with considerably reduced risk of complications.

It is a further object of the present invention to provide a device for holding an anastomotic site of a coronary artery motionless for an off-pump bypass operation without inviting deterioration of cardiac function during the operation caused by application of compression to the heart and without inviting scarring of the surface of the heart caused by application of the device.

It is a further object of the present invention to provide a practical device to hold an anastomotic site of the coronary artery motionless and bloodless during a bypass operation by restraining the bleeding from a dissected portion of the coronary artery for anastomosis.

Thus, it is an object of the present invention to provide an apparatus for temporarily immobilizing a local surgical site, such as an area of a beating heart, without requiring the use of stabilizing sutures.

It is a further object of the present invention to provide a disposable suction body for use in anastomosis of an autograft to the surface of a treatment site wherein the suction body includes a release mechanism so that the suction body can remain in place at an anastomotic site while the anastomosis is completed, and then the release mechanism can be actuated to remove the suction body from around the graft protruding from the anastomosis.

The suction body of the invention may be sized and adapted for attachment to the tip of an endoscopic catheter for stabilizing a treatment site located at the interior of the body to facilitate injection of a drug or obtaining a tissue sample from a moving bodily part.

These and other objectives are met with the features as defined in the claims. The present invention provides a suction body for temporarily immobilizing a local area of tissue. The invention flexible suction body comprises a suction channel containing a ring-shaped base defining an opening for exposing a treatment site, an outer flared, spreadable rim attached along the outer periphery of the base, a inner rim attached along the inner periphery of the base, a suction port for applying a partial vacuum within the suction channel, and at least one drain aperture in the suction body adapted for removing fluid from the treatment site while a partial vacuum is maintained in the suction channel. The suction device is coupled to a source of negative pressure.

In a preferred embodiment, the tip of the flared outer rim of the invention suction channel is sufficiently flexible that application of a partial vacuum to the suction body while the outer rim is in contact with the treatment site causes the outer, rim to flatten and spread outwardly across the surface of the treatment site. By this means a soft edge for the suction channel is created to avoid bruising of the underlying tissue. As the spreading of the outer rim also enlarges the suction field to apply the partial vacuum over an increased tissue area, localized hemorrhage (e.g., of capillaries) caused by the suction is minimized or avoided.

According to the invention the suction body is placed upon the surface of the treatment site. More specifically suction body is placed with the tip of the outer rim against the treatment site, and sufficient partial vacuum is applied to the suction channel via the suction port to cause the suction body to attach to the treatment site while the outer rim flattens and extends outwardly thereby stabilizing the treatment site. In one embodiment, the suction body further comprises a detachable handle attached to the exterior of the suction body wherein the handle is formable, but can be locked into a fixed orientation with respect to the suction body. In another embodiment, the invention suction body is sized for attachment to the tip of an endoscopic catheter, such as an injection catheter or a biopsy catheter.

### BRIEF DESCRIPTION OF THE DRAWINGS

The foregoing and other aspects of the present invention will best be appreciated with reference to the detailed description of the invention in conjunction with the accompanying drawings, wherein:
**Figure 1** is a perspective drawing showing the doughnut-shaped invention suction body mounted on the tines of a detachable handle.
**Figure 2** is a top plan drawing of the invention suction body.
**Figure 3** is a bottom plan drawing of the invention suction body.
**Figure 4** is a side view of the invention suction body as viewed from arrows 4-4 of Figure 2.
**Figure 5** is a cross-section drawing of the invention suction body taken through the section marked by arrows 5-5 of Figure 3.
**Figure 6** is a side view drawing of the invention suction body taken through the section marked by arrows 6-6in Figure 2.
**Figure 7** is a top plan view of the horseshoe-shaped invention suction body.
**Figure 8** is a bottom plan view of the horseshoe-shaped invention suction body.
**Figure 9** is a side view drawing of the horseshoe-shaped invention suction body as viewed from arrows 9-9 in Figure 7.
**Figure 10** is a drawing showing the invention suction body with detachable handle attached to an anastomotic site on the surface of a human heart.

### DESCRIPTION OF THE SPECIFIC EMBODIMENTS

The present invention provides a flexible suction body for stabilizing the surface of a treatment site made of a soft, compliant material, such as an elastomeric polymer, rather than hard plastic or rubber. Preferably, the suction body is made of a silicone, a urethane, or a mixture thereof, and the like. As the suction body is flexible, it will readily conform and cling to the shape of an uneven or contoured treatment site without application of compression on the treatment site while a partial vacuum is established and maintained within the suction channel.

The invention suction body comprises a suction channel containing a ring-shaped base defining an opening for exposing a treatment site, an outer flared, spreadable rim attached along the outer periphery of the base, an inner rim attached along the inner periphery of the base, a suction port for applying a partial vacuum within the suction channel, and at least one drain aperture in the suction body adapted for removing fluid from the treatment site while a partial vacuum is maintained in the suction channel. The suction channel has an overall shape and flexibility such that sufficient partial vacuum can be established and maintained therein to cause the suction body to fixedly cling to a treatment site when the suction body is placed and held against a treatment site while a partial vacuum is directed into the suction channel via the suction port, for example a partial vacuum in the range from about 100 mm Hg to about 600 mm Hg.

Generally, the suction body is flat in overall design rather than contoured, for example, with the base and tips of the inner and outer rims lying within planes. However, in an alternative embodiment, the suction body at the point of contact with the treatment surface can be contoured in overall shape to fit an uneven or contoured treatment site, such as the exterior of a human heart, and the like. Typical contoured shapes include convex, bowed, curved, and the like.

Preferably, the invention suction body further includes a release mechanism for opening and removing the suction body from a treatment site. The suction body is particularly adapted for attachment to an anastomotic site on the surface of a heart with the opening in the base shaped and sized to allow attachment of a coronary artery bypass graft to the anastomotic site therethrough. For example, the shape of the opening defined by the base of the suction body can be circular, elliptical, square, and the like. It is currently preferred that the base is ring-shaped, e.g., circular or elliptical. If circular, the opening generally has a diameter in the range from about 1mm to about 25mm, and the suction body is referred to as having the shape of a doughnut; and if elliptical, the opening generally has a shorter axis in the range from about 1mm to about 15 mm, and a longer axis in the range from about 2 mm to about 25 mm.

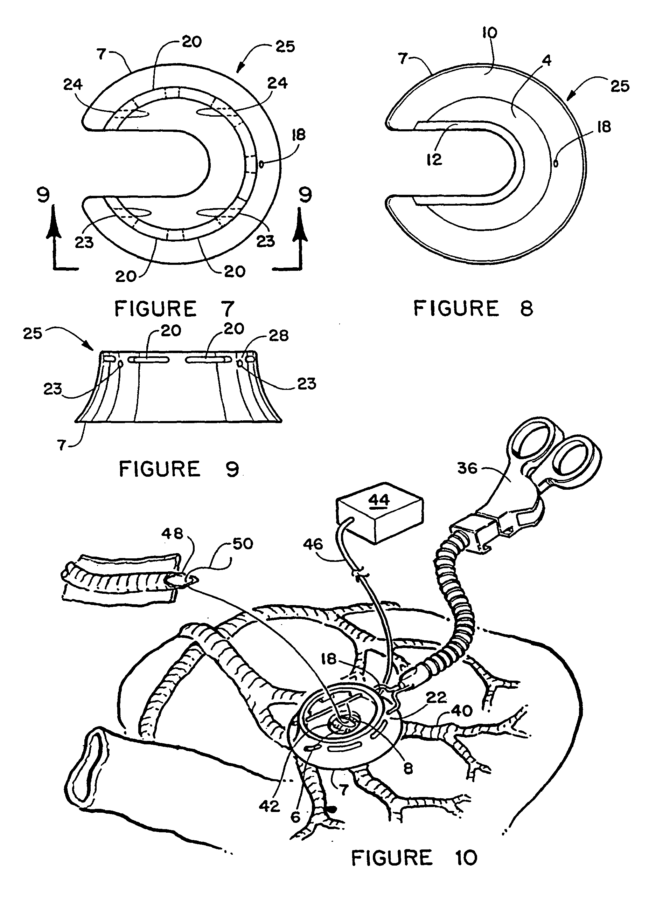

In an alternative embodiment the invention flexible suction body is in the overall shape of a horseshoe. In this embodiment the flexible suction body comprises a suction channel containing a horseshoe shaped base defining an opening for exposing a treatment site, a continuous rim running around the periphery of the base, a suction port for applying a partial vacuum within the suction channel, and at least one drain aperture in the suction body adapted for removing fluid from the treatment site while a partial vacuum is maintained in the suction channel, wherein the portion of the rim attached along the outer periphery of the base is flared, and spreadable. If the base is horseshoe shaped, the dimensions of the opening are generally similar to those created by an elliptical base, except that the ellipse is open at one end of the longer axis and the inner and outer rims of the suction channel merge around the ends of the horseshoe shape, shown as 25 in Figures 7-9.

To ensure that bruising or scarring caused by application of the device to the treatment site is minimized, the outer rim of the suction channel is highly flexible and compliant to the surface of the treatment site so that application of a partial vacuum to the suction body while the outer rim is in contact with the treatment site causes the outer rim to flatten and spread outwardly across the surface of the treatment site. This feature of the invention suction body is enhanced if the outer rim is substantially thinner at the tip than at the point of attachment to the base. Generally, the outer rim is graduated in thickness from the point of attachment to the base to the tip of the rim and the distance from the base to the tip is greater for the outer rim than for the inner rim so that, when the suction body is held against a treatment site with the tip of the outer rim against the treatment site and a partial vacuum is directed to the suction channel via the suction port, the suction developing in the suction channel pulls the suction body against the treatment site while the tip of the flexible outer rim flattens and spreads outwardly along the surface of the treatment site until the inner rim, which is generally less flexible and compliant than the tip of the outer rim, also comes into contact with the surface of the treatment site. This flattening and spreading of the highly flexible outer rim assures that the force of the suction body against the treatment site created by the partial vacuum within the suction channel is distributed over a much larger area of the suction body than if the outer rim were comparatively rigid and inflexible, thereby minimizing damage to blood capillaries and tissue at the treatment site.

The flattening and spreading outwardly of the outer rim as the suction body becomes attached to the treatment site also increases the area of the suction field appreciably over what it would be if the suction body were constructed of a non-compliant material, such as a hard plastic or hard rubber. The suction field of the invention suction body is generally in the range from about 0.1 cm² to about 10 cm², for example, about 3.5 cm² to about 6.0 cm².

The increased size of the suction field compared to that of prior art devices reduces the vacuum pressure applied to a given area of tissue by a given source of partial vacuum, thereby minimizing the risk that use of the invention suction body will cause localized suction hemorrhage, for example, to capillaries in the suction field. The vacuum pressure (i.e., per area of tissue) established by the invention suction body is generally in the range from about 100 mmHg to about 600 mm Hg, and preferably about 250 mmHg to about 450 mmHg, depending upon the size of the suction field and the vacuum pressure of the source of partial vacuum. In any event, the minimal vacuum pressure is over the systolic blood pressure of the subject.

In one embodiment presently preferred wherein the suction body is ring-shaped, the suction body includes a release mechanism for opening and removing the suction body from a treatment site, for example, a groove extending partially through the suction body such that opposing forces applied to the suction body on opposite sides of the groove will cause the suction body to part at the groove for removal from the treatment site, and the like. Whatever the design of the release mechanism, it is important that the overall design of the suction body and release mechanism be such that the release mechanism does not compromise the shape and integrity of the suction channel (i.e., its ability to maintain the desired vacuum pressure within the suction channel) until the release mechanism is actuated. Inclusion of a release mechanism in a suction body having a ring- shaped base provides the advantage that the suction body can remain in place at an anastomotic site while the anastomosis is completed and then the release mechanism can be actuated to remove the suction body from around a graft that protrudes through the ring-shaped opening in the suction body.

In another embodiment, presently preferred, the invention suction body further comprises a crown located along the outer periphery of the base on the side opposite the suction channel. The crown is relatively less flexible and compliant than the outer rim and is useful for providing a support into which is placed the at least one drain aperture, and preferably a plurality of drain apertures. For example, the plurality of drain apertures in the crown can be a plurality of slots at spaced intervals. The drain apertures allow for removal of fluid (e.g. blood) from the surface of the treatment site even while the suction body is attached thereto, for example, by directing a stream of gas (e.g., compressed air) into the opening in the suction body. The ability to remove fluid from the treatment site during the surgery being conducted within the opening enhances the surgeons' vision of the treatment site. Preferably, the suction body is also transparent or translucent to further enhance visibility of the treatment site while the suction body is attached thereto.

The crown is further useful for providing a support into which are placed one or more attachment sites for attaching a detachable handle to the exterior of the suction body. In a preferred embodiment, the handle is an instrument with attaching appendages, such as is known in the art as a flexible stabilizer (available from US Surgical under the name "mini CABG^{™} disposable stabilizer"), and the attachment sites are adapted to receive the attaching appendages of the flexible stabilizer. For example, if the attaching appendages are in the shape of a two-tined fork, the attachment sites are a plurality of apertures (e.g. drill holes) in the crown spaced for receiving the tines of the two-tined fork-like appendages on the flexible stabilizer. The detachable handle is useful for locating the suction body on a treatment site and for holding the suction body in place while the partial vacuum is directed into the suction channel and the vacuum builds up within the suction channel. In some embodiments, the handle is conformable (i.e., bendable) to assist in placing the suction body at a desired location on the treatment site, for example, on the surface of a beating heart, but can be locked into a fixed position once the suction body has been placed at a desired location. Optionally, once locked into fixed position, the handle can be temporarily attached to an exterior (e.g., stationary) object, such as an operating table or sternal or rib retractor, while suction is maintained to further stabilize the operative site and the suction body.

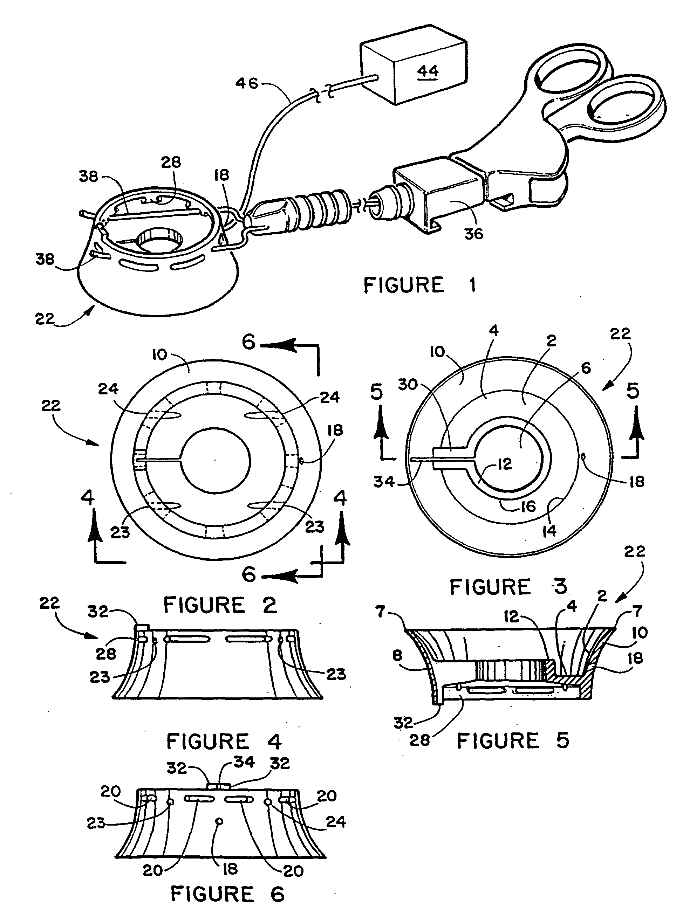

In one embodiment, the suction port is angled within the outer rim so that when the suction tube is attached thereto, the suction tube is directed away from the operative field for the convenience of the surgeons. For example, in one embodiment wherein the suction body is circular, the suction port within the outer rim is angled in two directions, to either side of a radius running through the center of the suction body, and downwardly as measured from the point of attachment of the outer rim to the base, as shown in Figure 5. In this way, when the tube for delivering a partial vacuum to the suction port is inserted into the suction port, the portion of the suction tube that extends from the suction body is directed away from the region of space where the surgeons' hands and instruments are working.

A preferred embodiment of the invention suction body is shown in Figures 1-6. In this embodiment suction body 22 is circular in overall shape and suction channel 2 is constructed of circular base 4 defining a circular opening 6 for exposing a treatment site, with outer flared, spreadable rim 10 attached along the outer periphery 14 of circular base 4, inner rim 12 attached along the inner periphery 16 of base 4, and suction port 18 located in the outer rim 10. Suction port 18 is adapted to receive suction tube 46, which is in communication with a source of partial vacuum 44 (i.e., negative pressure) and through which air is withdrawn from suction channel 2 to establish a partial vacuum therein.

Crown 28, a ridge encircling the outer periphery of base 4 on the side opposite to outer rim 10, provides a location for drain apertures 20 and attachment sites 23 and 24. For convenience, attachment sites 23 and 24 are situated symmetrically with respect to suction port 18. Suction port 18 is a small drill hole (approximately 1.5 mm in diameter) adapted for receiving the end of a suction tube, such as is found in hospital operating rooms. For example, the standard suction tube available in operating rooms has an outside diameter of 10 mm and an inner diameter of 6.5 -7.0 mm. An adapter as is known in the art can be used to sufficiently reduce the size of the standard tubing to establish fluid communication with the suction port in the suction body, which is generally substantially smaller. Optionally, a disposable suction reservoir, such as the Medi-vac^{™} system (Baxter Healthcare, Deerfield, IL), can be interposed between the suction source and the invention suction body to capture fluids collected by the suction body.

As shown in Figure 5, the suction port is angled to either side of a radius running through the center of the suction body, and downwardly as measured from the point of attachment of the outer rim to the base. As a result, when the suction tube 46 is attached to the suction port to direct a partial vacuum into suction channel 2, the suction tube extends from the suction body downwardly and to the side.

In this embodiment, suction channel 2 is not a complete toroidal space, but is interrupted by a reinforcing member 30 having the same height as the inner rim 12 (as measured from the base) and running between the flared outer rim 10 and the inner rim 12 along base 4. The vertical height of the flared outer rim (as measured from the plane of the base to that of the tip) is greater than that of the inner rim. The outer rim is also smoothly graduated in thickness with the point of attachment to the base being the thickest and the tip 7 being the thinnest. As further shown in Figure 10 when the suction body is held against a treatment site with the tip 7 of the outer rim against the treatment site and a partial vacuum is directed to the suction channel via the suction port, the partial vacuum developing in the suction channel pulls the suction body against the treatment site while the tip of the flexible outer rim flattens and spreads outwardly along the surface of the treatment site until the inner rim also comes into contact with the surface of the treatment site. Therefore, when attached by suction to the surface of the treatment site; the tip of the outer rim describes generally a circle of substantially greater circumference than that described by the outer rim before its attachment to the treatment site.

As shown in Figures 3, 5 and 6, the suction body includes a release mechanism consisting of a groove 34 having a width of about 0.25 mm to about 0.75 mm, which groove cuts through the suction body 22 and reinforcing member 30, except for a thin veneer 8 (e.g., about 0.1 mm at the point of least thickness) on the exterior of the outer rim that remains intact to preserve the integrity and shape of the suction channel. Release tabs 32 are located on the crown on either side of groove 34 and can readily be grasped by a forceps (or other functionally equivalent surgical instrument capable of applying opposite forces to the release tabs) so as to cause the suction body to part at groove 34 (i.e., by breaking through the thin veneer of the outer surface of the outer rim). An invention suction body constructed of elastomeric polymer having the design and release mechanism shown in Figures 3, 5 and 6 maintains the integrity of the suction channel when the suction body is applied to a treatment site and a partial vacuum of up to 600 mm Hg is applied to the suction port.

Figures 2, 4 and 6 show the location of attachment sites 23 and 24 in crown 28 of suction body 22 for receiving the attaching appendages of a detachable handle 36 with attaching appendages 38, shown here, respectively, as a flexible stabilizer with two attaching tines. The detachable handle attaches to the outer surface of the suction body by inserting the attaching appendages 38 into attachment sites 23 and 24. As shown here, attachment sites 23 and 24 are two sets of paired holes located in the crown with the members of each pair opposite one another so that each pair cooperatively receives one of the two tines (i.e., the attaching appendages).

The suction body of the invention may be sized and adapted for attachment to the distal tip of an endoscopic catheter, such as is used to deliver a therapeutic substance to an interior body site (e.g., a drug or gene therapy composition) or to obtain a diagnostic sample of tissue or fluid from a bodily surface, such as a solid tumor, as is known in the art. Exemplary catheters for use in connection with the invention suction device are disclosed in U.S. Patent Nos. 4,766,907, 5,626,609, 5,151,086, 5,420,698, 5,441,507. Many such catheters have a needle or trocar slidably mounted within the distal tip of the catheter such that once the catheter is in the desired location at an interior body site, the operator can cause the needle or trocar to penetrate into the bodily surface at the treatment site either to inject a substance (e.g. a therapeutic drug) or to retrieve a substance therefrom (e.g. a solid tumor sample for histological analysis). Thus, in the endoscopic suction body, the opening in the base of the suction channel is sized to passage therethrough of a needle or trocar from the interior of the endoscopic catheter into the treatment site.

In such therapeutic or diagnostic endoscopic procedures, it is important that the needle or trocar penetrate the surface of the treatment site to a precise depth. When the treatment site is a moveable interior body site, such as a heart, stomach, esophagus or lung, it is difficult to precisely control the depth of penetration of the needle or trocar into the treatment site. This problem can be overcome by attaching an appropriately sized invention suction body to the distal tip of the endoscopic catheter (e.g. with the suction tube running up the length of a lumen within the catheter) prior to insertion of the catheter into the interior body site. Once the catheter is inserted into the interior body site and the suction body is moved into contact with the desired treatment site, the vacuum is applied to the suction channel in the suction body as described herein. The suction body clings to the treatment site so as to hold the distal tip against the treatment site with sufficient stability to ensure that actuation of the needle or trocar within the endoscopic catheter will inject the drug to the desired depth or cause the trocar to obtain a sample from a predetermined depth within the treatment site. Thus, as applied to the endoscopic suction body of the invention, the term "stabilize" means that the tip of the endoscopic catheter is firmly held against the treatment site, even if the treatment site is in motion, rather than meaning that the suction body exerts sufficient control over the movement of the treatment site to actually minimize the movement of a moving treatment site.

As the negative pressure developed in the suction body is inversely proportional to the suction surface of the suction body at any given suction pressure, it can be seen that the miniature size of the invention endoscopic suction body will, however, cause a much higher suction pressure to develop within the suction body than would be suitable for use with a larger sized suction body, for example in a suction body sized for attachment to an anastomotic site on the surface of the heart. Thus, in the endoscopic suction body of the invention, the flared, spreadable outer rim is particularly useful for preventing unwanted damage to the treatment site.

The methods, which are not forming part of the invention, for stabilizing a treatment site utilizing the invention suction bodies are now described. The methods comprise placing a suction body upon the surface of the treatment site, wherein the suction body comprises:
a suction channel comprising:
   a ring- or horseshoe-shaped base defining an opening for exposing a treatment site,
   an outer flared, spreadable rim attached along the outer periphery of the base,
   an inner rim attached along the inner periphery of the base,
   a suction port for establishing a partial vacuum within the suction channel, and
   at least one drain aperture in the suction body adapted for removing fluid from the treatment site while a partial vacuum is maintained in the suction channel,
wherein the suction body is placed with the tip of the outer rim against the treatment site, and
applying sufficient partial vacuum to the suction channel via the suction port to cause the suction body to cling to the treatment site, thereby stabilizing the treatment site. Due to the flexible construction of the suction body, especially of the outer rim, as the partial vacuum becomes established within the suction channel due to application of the partial vacuum to the suction channel, the outer rim of the suction body flattens and spreads outwardly.

In a presently preferred embodiment, the holding of the suction body is accomplished by the surgeon grasping a formable detachable handle attached to the exterior of the suction channel, such as the type of instrument that is known in the art as a flexible stabilizer (available from US Surgical under the name "mini CABG^{™} stabilizer"). Preferably, the method further comprises locking the handle into a fixed orientation with respect to the suction body for holding while the partial vacuum is applied to the suction channel to cause the suction body to cling to the treatment site. Optionally, the handle can also be temporarily affixed to an exterior object, such as an operating table or a rib retractor, and the like, to further stabilize the suction body throughout the operation. In this preferred embodiment of the invention, the handle attached to the suction body is not used to pull up on the treatment site because it has been discovered that pulling the treatment site away from underlying tissue places sufficient stress on a delicate treatment site to permanently damage tissue. It is a particular advantage of the invention that a delicate treatment site, such as the surface of a beating heart, can be stabilized using the described methods and invention suction body without pulling the surface of the treatment site away from muscular contractions deeper within the underlying heart musculature.

The invention is now illustrated with respect to a representative OPCAB surgery as shown in Figure 10. First, the invention suction body 22 is positioned and held at an anastomotic site 8 of the coronary artery 40 using a detachable handle 36, shown here as a flexible stabilizer, attached to the exterior of suction body 22 in such a manner that the anastomotic site can be viewed through circular opening 6, which is surrounded by suction channel 2. Care is taken in placing the suction body that both rims of the suction channel cross over both the proximal side and the distal side of the coronary artery 40. While the suction body is held in position against the surface of the heart, a suction tube 46 attached to a suction source 44 is attached to the suction port of the suction body until sufficient partial vacuum is established in the suction channel to cause the suction body to cling to the surface of the beating heart so as to stop the blood flow in the artery within opening 8 without the need to compress the beating heart. Concurrently, the treatment site will be stably held despite the motion of the beating heart without pulling up on the handle attached to the suction body to raise the treatment site. In fact, due to the suction force applied to the treatment site by the suction body, the anastomotic site will be somewhat raised within opening 8 (i.e., partially extruded therethrough).

Next, an aperture 42 is made with a scalpel on the portion of coronary artery 40 exposed within opening 8 and an internal thoracic artery 48 (or a radial artery, inferior gastroepiploic artery or saphenous vein) is 1ed to aperture 42 to be anastomosed to the coronary artery with a suture 50, as shown in Figure 10. Meanwhile, internal thoracic artery 48 is occluded at a distal portion (not shown) to hamper the blood flow in artery 48 during formation of the anastomosis. When the anastomosis has been securely formed, the pressure within the suction channel is returned to atmospheric pressure, for example, by disengagement of suction tube 46 from suction port 18, and the suction body is detached from the surface of the heart and from coronary artery 40 so that blood flow in artery 40 resumes. Blood flow within internal thoracic artery 48 is also resumed, flowing through the distal side of coronary artery 40. Once blood flow has been reestablished, the release mechanism in the suction body is actuated by grasping (e.g. with forceps) release tabs 32 located on the crown 28 on either side of groove 34 as shown in Figures 2, 3 and 5, and applying opposite forces to the release tabs to cause the suction body to part at groove 34. The surgery ends with the final step of suturing the chest closed.

OPCAB surgery utilizing the suction body according to the invention can be performed in about 90 to 180 minutes, on average, from the thoracotomy through the suturing closed of the chest so that the fatigue of the patient as well as the burden on the surgeons and their assistants, such as the nurses, can be greatly reduced.

To further facilitate the surgery, the invention may be used in a method further comprising removing any fluid, such as blood, that accumulates within the ring-shaped opening of the suction body at the treatment site without removal of the suction body by removing such fluid through the at least one drain aperture in the suction body, for example, by applying a stream of gas to the treatment site to blow the fluid through the drain aperture and away from the treatment site.

In the invention, the suction body is used to apply a partial vacuum over a surface area in the range from about 0.1 cm² to about 10 cm², and preferably from about 3.5 cm² to about 6.0 cm² so as to minimize formation of localized suction hemorrhage on the surface of the treatment site.

In use, a suitably sized suction body according to the invention is attached to the distal tip of an endoscopic catheter, such as is used to deliver a therapy to an interior body site or to obtain diagnostic information from the body site. For example, the suction body can be used to hold the tip of a fiber optic catheter against a treatment site while a laser or other therapeutic or diagnostic light is applied to the surface of the treatment site through the opening in the base of the invention suction body. Alternatively, an invention suction body can be attached to the distal tip of a catheter having a needle or trocar slidably mounted within the distal tip of the catheter for delivering or retrieving a substance below the surface of the treatment site. Once the catheter is manipulated to place the suction body at a desired location at an interior body site and suction is applied to cause the suction body to cling to the surface of the treatment site, the operator can cause the needle or trocar to penetrate into the bodily surface at the treatment site either to inject a substance (e.g: a therapeutic drug) or to retrieve a substance therefrom (e.g. a solid tumor sample for histological analysis) via the opening in the suction body. Because the catheter is firmly attached to the treatment site through the action of the suction body, the needle or trocar will penetrate into the treatment site for a predetermined depth of penetration even if the treatment site is located on a bodily surface that is in motion.

This embodiment of the invention affords the advantage that the needle or trocar from the catheter will attain a predetermined depth of penetration, thereby enhancing the reliability of the information derived from a diagnostic sample retrieved or assuring that a drug, such as an angiogenesis drug (i.e. a drug suitable for enhancing development of new blood vessels in the heart), is delivered to the most suitable depth for achieving the therapeutic goal.

It will be apparent to those skilled in the art that various changes may be made in the invention without departing from the scope of the invention as defined by the appended claims.

## Claims

1. A flexible suction body (22) for stabilizing the surface of a treatment site, said suction body comprising:
a suction channel (2) comprising:
a ring-or horseshoe-shaped base (4) defining an opening (6) for exposing a treatment site,
an outer flared, spreadable rim (10) attached along the outer periphery (14) of the base,
an inner rim (12) attached along the inner periphery (16) of the base,
a suction port (18) for establishing a partial vacuum within the suction channel **characterized by**:
a crown (28) in the form of a ridge encircling the outer periphery of a side of the base opposite the suction channel, wherein the crown has at least two apertures (23, 24) for receiving insertable times of a stabilizer device (36).

2. The suction body according to claim 1 wherein the suction body is ring-shaped further comprising a release mechanism (34) for opening and removing the suction body from a treatment site.

3. The suction body according to claim 2 wherein the release mechanism comprises a groove (34) extending partially through the suction body such that opposing forces applied to the suction body on opposite sides of the groove will cause the suction body to part at the groove for removal from the treatment site.

4. The suction body according to claim 3 wherein the groove does not compromise the integrity of the suction channel.

5. The suction body according to claim 1 wherein the treatment site is an anastomotic site (8) on the surface of a heart and the opening in the base is sized to allow attachment of a coronary artery bypass graft to the anastomotic site therethrough.

6. The suction body according to claim 1 wherein the ring-shaped base is circular defining a circular opening.

7. The suction body according to claim 6 wherein the diameter of the circular opening is in the range from about 1mm to about 25mm.

8. The suction body according to claim 6 wherein the diameter of the circular opening is in the range from about 15mm to about 20mm.

9. The suction body according to claim 5 wherein the ring-shaped base is elliptical defining an elliptical opening in the base.

10. The suction body according to claim 9 wherein the shorter axis of the elliptical opening is in the range from about 1mm to about 20 mm, and the longer axis of the elliptical opening is in the range from about 2 mm to about 25 mm.

11. The suction body according to claim 1 wherein the outer rim is graduated in thickness from the point of attachment to the base to the tip of the rim and the vertical distance from the base to the tip for the outer rim is greater than for the inner rim.

12. The suction body according to claim 1 wherein the base and suction channel are horseshoe-shaped.

13. The suction body according to claim 1 wherein the tip of the outer rim is sufficiently flexible that application of a partial vacuum to the suction body while the outer rim is in contact with the treatment site causes the outer rim to flatten and spread outwardly across the surface of the treatment site.

14. The suction body according to claim 1 wherein the suction field of the suction device has an area in the range from about 0.1 cm² to about 10 cm².

15. The suction body according to claim 1 wherein the suction body is fabricated from an elastomeric polymer.

16. The suction body according to claim 15 wherein the elastomeric polymer is a silicon or a urethane.

17. The suction body according to claim 1 wherein the suction body further comprises at least one drain aperture (20) adapted for removing fluid from the treatment site while a partial vacuum is maintained in the suction channel.

18. The suction body according to claim 1 wherein the suction body further comprises a plurality of drain apertures (20) located in the crown adapted for removing fluid from the treatment site while a partial vacuum is maintained in the suction channel.

19. The suction body according to claim 1 wherein the suction channel extends only partially around the periphery of the base.

20. The suction body according to claim 1 wherein the suction port is located within the outer rim.

21. The suction body according to claim 20 wherein the suction port is angled downwardly and sidewards from the point of attachment of the outer rim to the base.

22. The suction body according to claim 1 wherein the suction body is transparent or translucent.

23. The suction body according to claim 1 wherein the suction body is contoured.

## Patentansprüche

1. Flexibler Saugkörper (22) zur Stabilisierung der Oberfläche einer Behandlungsstelle, wobei der Saugkörper aufweist:
einen Saugkanal (2), der aufweist:
eine ring- oder hufeneisenförmige Basis (4), die eine Öffnung (6) zum Freilegen einer Behandlungsstelle definiert,
einen konisch erweiterten, ausdehnbaren Außenkranz (10), der längs des Außenumfangs (14) der Basis befestigt ist,
einen Innenkranz (12), der längs des Innenumfangs (16) der Basis befestigt ist,
einen Sauganschluß (18) zur Herstellung eines Unterdrucks im Saugkanal, **gekennzeichnet durch**:
eine Krone (28) in der Form einer Rippe, die den Außenumfang einer Seite der Basis gegenüberliegend zum Saugkanal umgibt, wobei die Krone mindestens zwei Öffnungen (23,24) zur Aufnahme einsetzbarer Zinken einer Stabilisatorvorrichtung (36) aufweist.

2. Saugkörper nach Anspruch 1, wobei der Saugkörper ringförmig ist und ferner einen Lösungsmechanismus (34) zur Öffnung und Entfernung des Saugkörpers von einer Behandlungsstelle aufweist.

3. Saugkörper nach Anspruch 2, wobei der Lösungsmechanismus eine Nut (34) aufweist, die sich teilweise durch den Saugkörper erstreckt, so daß entgegengesetzte Kräfte, die auf gegenüberliegenden Seiten der Nut auf den Saugkörper ausgeübt werden, bewirken werden, daß sich der Saugkörper an der Nut zur Entfernung von der Behandlungsstelle teilt.

4. Saugkörper nach Anspruch 3, wobei die Nut die Integrität des Saugkanals nicht beeinträchtigt.

5. Saugkörper nach Anspruch 1, wobei die Behandlungsstelle eine Anastomosenstelle (8) auf der Oberfläche eines Herzens ist und die Öffnung in der Basis bemessen ist, dort hindurch eine Befestigung eines Koronararterien-Bypasstransplantats an der Anastomosenstelle zu ermöglichen.

6. Saugkörper nach Anspruch 1, wobei die ringförmige Basis kreisförmig ist, wobei eine kreisförmige Öffnung definiert wird.

7. Saugkörper nach Anspruch 6, wobei der Durchmesser der kreisförmigen Öffnung im Bereich von etwa 1 mm bis etwa 25 mm liegt.

8. Saugkörper nach Anspruch 6, wobei der Durchmesser der kreisförmigen Öffnung im Bereich von etwa 15 mm bis etwa 20 mm liegt.

9. Saugkörper nach Anspruch 5, wobei die ringförmige Basis elliptisch ist, wobei eine elliptische Öffnung in der Basis definiert wird.

10. Saugkörper nach Anspruch 9, wobei die kürzere Achse der elliptischen Öffnung im Bereich von etwa 1 mm bis etwa 20 mm liegt, und die längere Achse der elliptischen Öffnung im Bereich von etwa 2 mm bis etwa 25 mm liegt.

11. Saugkörper nach Anspruch 1, wobei die Dicke des Außenkranzes von der Befestigungsstelle an der Basis zur Spitze des Kranzes abgestuft ist und der vertikale Abstand von der Basis zur Spitze für den Außenkranz größer als für den Innenkranz ist.

12. Saugkörper nach Anspruch 1, wobei die Basis und der Saugkanal hufeneisenförmig sind.

13. Saugkörper nach Anspruch 1, wobei die Spitze des Außenkranzes ausreichend flexibel ist, so daß die Ausübung eines Unterdrucks auf den Saugkörper, während sich der Außenkranz mit der Behandlungsstelle in Kontakt befindet, bewirkt, daß sich der Außenkranz abflacht und sich über die Oberfläche der Behandlungsstelle nach außen ausdehnt.

14. Saugkörper nach Anspruch 1, wobei das Sauggebiet der Saugvorrichtung eine Fläche im Bereich von etwa 0,1 cm² bis etwa 10 cm² aufweist.

15. Saugkörper nach Anspruch 1, wobei der Saugkörper aus einem Elastomerpolymer gefertigt ist.

16. Saugkörper nach Anspruch 15, wobei das Elastomerpolymer aus Silikon oder Urethan besteht.

17. Saugkörper nach Anspruch 1, wobei der Saugkörper ferner mindestens eine Ableitungsöffnung (20) aufweist, die zur Entfernung einer Flüssigkeit von der Behandlungsstelle eingerichtet ist, während ein Unterdruck im Saugkanal aufrechterhalten wird.

18. Saugkörper nach Anspruch 1, wobei der Saugkörper ferner mehrere Ableitungsöffnungen (20) aufweist, die sich in der Krone befinden, die zur Entfernung einer Flüssigkeit von der Behandlungsstelle eingerichtet sind, während ein Unterdruck im Saugkanal aufrechterhalten wird.

19. Saugkörper nach Anspruch 1, wobei sich der Saugkanal nur teilweise um den Umfang der Basis erstreckt.

20. Saugkörper nach Anspruch 1, wobei der Sauganschluß im Außenkranz angeordnet ist.

21. Saugkörper nach Anspruch 20, wobei der Sauganschluß von der Befestigungsstelle des Außenkranzes an der Basis nach unten und seitwärts angewinkelt ist.

22. Saugkörper nach Anspruch 1, wobei der Saugkörper durchsichtig oder durchscheinend ist.

23. Saugkörper nach Anspruch 1, wobei der Saugkörper konturiert ist.

## Revendications

1. Corps d'aspiration flexible (22) destiné à stabiliser la surface d'une zone de traitement, ledit corps d'aspiration comprenant :
un canal d'aspiration (2) comprenant :
une base en forme d'anneau ou de fer à cheval (4) définissant une ouverture destinée à exposer une zone de traitement,
une collerette extérieure évasée extensible (10) fixée le long de la périphérie extérieure (14) de la base,
une collerette intérieure (12) fixée le long de la périphérie intérieure (16) de la base,
un port d'aspiration (18) destiné à établir un vide partiel à l'intérieur du canal d'aspiration,
**caractérisé par**
une couronne (28) ayant la forme d'une arrête qui encercle la périphérie extérieure d'un côté de la base opposé au canal d'aspiration, dans laquelle la couronne possède au moins deux ouvertures (23, 24) destinées à recevoir des dents pouvant être insérées d'un dispositif de stabilisateur (36).

2. Corps d'aspiration selon la revendication 1, dans lequel le corps d'aspiration a la forme d'un anneau et comprend en outre un mécanisme de libération (34) destiné à ouvrir et à retirer le corps d'aspiration de la zone de traitement.

3. Corps d'aspiration selon la revendication 2, dans lequel le mécanisme de libération comprend une rainure (34) s'étendant partiellement à travers le corps d'aspiration de telle sorte que des forces opposées appliquées au corps d'aspiration sur les côtés opposés de la rainure entraîneront le détachement du corps d'aspiration au niveau de la rainure afin de le retirer de la zone de traitement.

4. Corps d'aspiration selon la revendication 3, dans lequel la rainure ne compromet pas l'intégrité du canal d'aspiration.

5. Corps d'aspiration selon la revendication 1, dans lequel la zone de traitement est une zone anastomotique (8) sur la surface d'un coeur et l'ouverture dans la base est dimensionnée de manière à permettre la fixation d'un greffon de pontage d'artère coronaire à la zone anastomotique à travers cette dernière.

6. Corps d'aspiration selon la revendication 1, dans lequel la base en forme d'anneau est circulaire et définit une ouverture circulaire.

7. Corps d'aspiration selon la revendication 6, dans lequel le diamètre de l'ouverture circulaire est dans la plage d'environ 1 mm à environ 25 mm.

8. Corps d'aspiration selon la revendication 6, dans lequel le diamètre de l'ouverture circulaire est dans la plage d'environ 15 mm à environ 20 mm.

9. Corps d'aspiration selon la revendication 5, dans lequel la base en forme d'anneau est elliptique et définit une ouverture elliptique dans la base.

10. Corps d'aspiration selon la revendication 9, dans lequel l'axe le plus court de l'ouverture elliptique est dans la plage d'environ 1 mm à environ 20 mm et l'axe le plus long de l'ouverture elliptique est dans la plage d'environ 2 mm à environ 25 mm.

11. Corps d'aspiration selon la revendication 1, dans lequel la collerette extérieure a une épaisseur échelonnée depuis le point de fixation à la base jusqu'à la pointe de la collerette et la distance verticale depuis la base jusqu'à la pointe de la collerette extérieure est supérieure à celle de la collerette intérieure.

12. Corps d'aspiration selon la revendication 1, dans lequel la base et le canal d'aspiration ont la forme d'un fer à cheval.

13. Corps d'aspiration selon la revendication 1, dans lequel la pointe de la collerette extérieure est suffisamment flexible pour que l'application d'un vide partiel au corps d'aspiration lorsque la collerette extérieure est en contact avec la zone de traitement amène la collerette à s'aplatir et à s'étaler vers l'extérieur à travers la surface de la zone de traitement.

14. Corps d'aspiration selon la revendication 1, dans lequel le domaine d'aspiration du dispositif d'aspiration possède une surface dans la plage d'environ 0,1 cm² à environ 10 cm².

15. Corps d'aspiration selon la revendication 1, dans lequel le corps d'aspiration est fabriqué à partir d'un polymère élastomère.

16. Corps d'aspiration selon la revendication 15 dans lequel le polymère élastomère est une silicone ou un uréthane.

17. Corps d'aspiration selon la revendication 1, dans lequel le corps d'aspiration comprend en outre au moins une ouverture de drainage (20) adaptée pour éliminer le fluide provenant de la zone de traitement tandis qu'un vide partiel est maintenu dans le canal d'aspiration.

18. Corps d'aspiration selon la revendication 1, dans lequel le corps d'aspiration comprend en outre une pluralité d'ouvertures de drainage (20) situées dans la couronne et adaptées pour éliminer le fluide provenant de la zone de traitement tandis qu'un vide partiel est maintenu dans le canal d'aspiration.

19. Corps d'aspiration selon la revendication 1, dans lequel le canal d'aspiration s'étend uniquement partiellement autour de la périphérie de la base.

20. Corps d'aspiration selon la revendication 1, dans lequel le port d'aspiration est situé à l'intérieur de la collerette extérieure.

21. Corps d'aspiration selon la revendication 20, dans lequel le port d'aspiration est incliné vers le bas et vers le côté depuis le point de fixation de la collerette extérieure jusqu'à la base.

22. Corps d'aspiration selon la revendication 1, dans lequel le corps d'aspiration est transparent ou translucide.

23. Corps d'aspiration selon la revendication 1, dans lequel le corps d'aspiration est profilé.
